# EUROPEAN PATENT APPLICATION

(11) **EP 3 231 305 A1**
(43) Date of publication of application: **18.10.2017**
(21) Application number: 15868084.3
(22) Date of filing: 11.12.2015
(51) Int. Cl.: A41F 9/02, A61B 5/053

(54) **FITTING DEVICE FOR ADJUSTING ELECTRODE BELTS**

(30) Priority: 12.12.2014 BR 102014031272
(71) Applicant: Timpel S.A., 05417-020 São Paulo - SP (BR)
(72) Inventor: HOLZHACKER, Rafael, 05417-020 São Paulo - SP (BR); CANDIANI, Igor Nowaski, 05711-000 São Paulo - SP (BR)
(74) Representative: Schwabe - Sandmair - Marx
(86) International application number: PCT/BR2015/050247
(87) International publication number: WO 2016/090451

(57) **Abstract**

The present invention refers to a shapewear device for the adjustment of an electrode belt around the body of a human or animal patient. The device of the present invention comprises: one tensioner body (3); at least one first arm (2A) and one second arm (2B); both arms (2A, 2B) stretch out from the tensioner body (3) in opposite directions, each arm (2A, 2B) has a free end (7A, 7B) comprising at least one mean of fastening (4) to an electrode belt.

## Description

### FIELD OF THE INVENTION

The present invention relates generally to a shapewear device for the adjustment of an electrode belt around the body of a human or animal patient.

### BACKGROUND OF THE INVENTION

The electrical impedance tomography (EIT) is an image acquisition technique based on the application of alternating electrical signals, on the surface of patient's body, with frequencies between 10 kHz and 2.5 MHz. The equipment used for this purpose comprises a plurality sensors (electrodes) placed on the skin. They are connected, by means of electrical conductors, to a processing unit that makes the mentioned alternating signal. The method used has several steps. In each of the steps a pair of electrodes is selected for the injection of the signal mentioned, while the induced voltage is measured, which is acquired by the electrodes that were not selected. On the following steps, other pairs of electrodes are selected for the injection of the signal, continuing this sequence until all electrodes from the equipment have been selected, completing one exploring cycle. The induced tensions that were acquired by the electrodes undergo a specific software treatment, allowing the image generation that usually represents the ventilation and perfusion phenomena in the organism observed.

Normally, the electrodes are held by a belt, which is placed around the body of the patient, preferably in the thorax area. In this regard, it is relevant to mention the document PI 0704408-9 that describes modular belts that have multiple electrodes, to be applied around a part of the body of a human or animal patient.

Today, doctors, patients and nurses have been noticing that the electrode belts do not properly fit to the body of all patients, leaving gaps. With these gaps, the electrodes are likely to me misplaced on the patient's thorax, leading to flaws on the electrical contact. Consequently, the reading of the electrical impedance signals acquired may carry mistakes.

The gaps mentioned are mainly consequence of the shape of the human thorax, which has at least two main concavities, namely: on the area of the sternum bone (front part of the thorax); and around the vertebral column. Evidently, other areas of thorax of a certain patient may also create gaps, the former, however, are the most common.

This way, the need for the development of a technical solution for this problem arises.

Therefore, to solve the problems exemplified above and other existing problems with the state of the art, one of the present invention's objective is to offer a shapewear device for the adjustment of an electrode belt around the body of a patient that allows the tensioning of the electrode belt in a way that eliminates the gaps.

The present invention, by means of its own characteristics, may, still, solve other problems of the state of the art that are not brought up here for example, once the repertoire here discussed regarding electrode belts and its problems is exemplary and non-exhausting.

### DESCRIPTION OF THE INVENTION

In order to avoid the inconveniences of the state of the art above mentioned, among others, the present invention is a shapewear device for the adjustment of an electrode belt that comprises: a tensioner body, at least a first arm and a second arm; both arms stretch out from the tensioner body in opposite directions, each arm has a free end and each free end has at least one mean of fastening it to an electrode belt.

According to the additional and/or alternative embodiments of the present invention, the following characteristics, and its possible variations, may also be present, alone or combined:
- the tensioner body comprises a bulged profile;
- the tensioner body has a protruding central portion and at least two side portions opposite one another and in relation to the central portion, wherein the lateral portions have less protrusion in comparison to the central portion;
- each arm stretches out from a lateral of the protruding body;
- the tensioner body comprises a deformable material wrapped by a casing of non-woven fabric.
- the arms are made of non-woven fabric; and
- the means of fastening an electrode belt comprise a double-sided hook tape.

### BRIEF DESCRIPTION OF THE DRAWINGS

The objectives, functional improvements and advantages of the shapewear device, object of the present invention, will be clear to those skilled in the art from the description below made in regard to a preferred embodiment, which makes references to the attached figures. The figures are schematic, and its dimension or proportion may not correspond to reality, once they only aim at describing the invention didactically.
FIG. 1 illustrates a perspective view of the shapewear device of the present invention;
FIG. 2 illustrates a side elevational view of the shapewear device of the present invention; and
FIG. 3 illustrates a perspective view of an alternative embodiment of the present invention;

### DESCRIPTION OF THE PREFERRED EMBODIMENT

The invention is now described regarding its preferred embodiments, referring to the attached figures. In the following figures and description, similar parts are marked throughout the descriptive report and figures with the same reference numbers. The figures are not necessarily in scale. Certain characteristics of the invention may be shown with exaggeration of scale or schematically, and some details of conventional elements may not be shown in order to illustrate this description in a more clear and concise way. The present invention is sensitive to embodiments carried in different ways. Specific embodiments are described in details and shown in the figures, with the understanding that the description must be considered an example of its principles, and the purpose is not to limit the invention only to what is illustrated and described in the present descriptive report. We must acknowledge that the different teachings of the embodiments discussed next may be separately employed or in any appropriate combination to provide the same results desired.

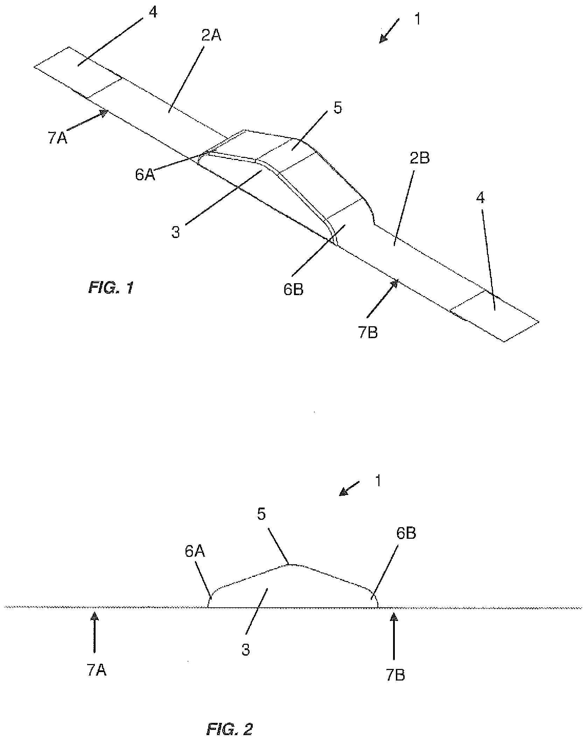

As it may be seen from FIG. 1, the shapewear device 1 of the present invention comprises a tensioner body 3, a first arm 2A and a second arm 2B. The tensioner body 3 has a bulged profile, formed by a protruding central portion 5 and two lateral portions 6A and 6B that have less protrusion in comparison to the central portion 5. Besides, the figures show that the lateral portions 6A and 6B are opposite one another and in relation to the central portion 5.

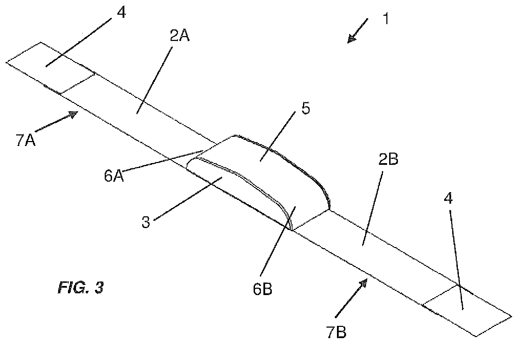

In an alternative performance of the present invention, shown on FIG. 3, the tensioner body 3 also has a protruding central portion 5 and at least two lateral portions 6A, 6B opposite one another in relation to the central portion 5. The lateral portions 6A, 6B, however, have essentially the same protrusion in comparison to the central portion 5.

In the preferred embodiment represented in the figures, the tensioner body 3 has the shape of a substantially elongated block; in preferred embodiments, however, the tensioner body 3 may carry other shapes. For example, the tensioner body 3 could be substantially round, having a protruding central area and at least two less protruding side areas, that is, "lower", in comparison to the central portion. Various sizes of the tensioner body 3 and arms 2A and 2B are also possible.

As it is seen on the figures, the arms 2A and 2B stretch out from the tensioner body 3 in opposite directions and, more precisely, each arm 2A and 2B stretches out from a lateral portion 6A, 6B of the protruding body 3. Still, each of the arms 2A, 2B have a free end 7A and 7B, wherein each free end 7A and 7B comprises means of fastening 4 to an electrode belt. There are several means of fastening within the scope of the present invention; nonetheless, just to draw an example, the means of fastening may consist of a hook tape or even adhesives.

Regarding the material used for the production of the present invention's shapewear device, the tensioner body 3 may be made of a deformable material enveloped by a casing made of a material that is not aggressive to the patient's body. In the preferred embodiment here exemplified, the tensioner body 3 is made of foam and its casing of non-woven fabric. The arms 2A and 2B, in turn, are also made of non-woven fabric in the preferred embodiment of the present invention. In one embodiment of the present invention the arms 2A and 2B may be made of one single strip of non-woven fabric, over which the inferior part of the tensioner body 3 is placed, which, then, is enveloped with more strips of the non-woven fabric, interconnected, in order to make the casing on its body.

As it is possible to notice on FIGS. 1 and 3, the means of fastening 4 are placed on the upper face of the free ends 7A, 7B. According to this layout, the tensioner body 3 is placed on an electrode belt already put around a patient. The position of the tensioner body 3 is such that its central portion 5 faces the electrode belt against the patient's body in an area where there is a gap between the belt and the body. After pressing the central portion 5 against the belt and, consequently, against the gap, keeping the belt tight on the patient's body, the free ends 7A and 7B of the shapewear device are fastened on the belt, by the means of fastening 4. The electrode belt can still be placed inside a non-woven fabric casing, to make its contact with the patient's body more comfortable. In this case, the means of fastening 7A and 7B must be specific for the fastening on this material of the shapewear device 1 of the present invention, such as the already mentioned "hook or loop" tape.

This way, the present invention, by means of its unique and innovative features, fills the existing gap in the state of the art, solving technical problems, among which those related to the existing gap between the patient's body and the electrode belt of electrical impedance tomography.

Despite the shapewear device being especially useful to tightly fasten an electrode belt around a patient's body, the present invention can be implemented in other ways of applications and it may show modifications in the manner it is implemented, so that the scope of protection of the invention is only limited by the content of the following claims, including the possible equivalent variations.

## Claims

1. SHAPEWEAR DEVICE FOR THE ADJUSTMENT OF AN ELECTRODE BELT, wherein the device comprises: one tensioner body (3); at least one first arm (2A) and one second arm (2B); both arms (2A, 2B) stretch out from the tensioner body (3) in opposite directions, each arm (2A, 2B) having a free end (7A, 7B) comprising at least one mean of fastening (4).

2. SHAPEWEAR DEVICE of claim 1, wherein the tensioner body (3) comprises a bulged profile.

3. SHAPEWEAR DEVICE of claim 2, wherein the tensioner body (3) has a protruding central portion (5) and at least two lateral portions (6A, 6B) opposite one another and in relation to the central portion (5), wherein the lateral portions (6A, 6B) have less protrusion in comparison to the central portion (5).

4. SHAPEWEAR DEVICE of claim 2, wherein the tensioner body (3) has a protruding central portion (5) and at least to lateral portions (6A, 6B) opposite one another and in relation to the central portion (5), wherein the lateral portions (6A, 6B) have equal protrusion in comparison to the central portion (5).

5. SHAPEWEAR DEVICE of claims 1, 3 and 4, wherein each arm (2A, 2B) stretches out from a lateral portion (6A, 6B) of the protruding body (3).

6. SHAPEWEAR DEVICE of claim 1, wherein the tensioner body (3) comprises a deformable material enveloped by a non-woven fabric casing.

7. SHAPEWEAR DEVICE of claim 1, wherein the arms (2A, 2B) are made of non-woven fabric.

8. SHAPEWEAR DEVICE from claim 1, wherein the means of fastening (4) on an electrode belt comprise a hook or loop tape.
